# EUROPEAN PATENT APPLICATION

(11) **EP 4 726 384 A1**
(43) Date of publication of application: **15.04.2026**
(21) Application number: 24819380.7
(22) Date of filing: 06.06.2024
(51) Int. Cl.: G01N 27/62, G01N 30/72

(54) **METHOD FOR DETECTING SOYBEAN USING MASS SPECTROMETER**

(30) Priority: 07.06.2023 JP 2023094352
(71) Applicant: NISSIN FOODS HOLDINGS CO., LTD., Yodogawa-ku Osaka-shi, Osaka 532-8524 (JP)
(72) Inventor: ITO, Mina, Osaka-shi Osaka 532-8524 (JP); MIYAZAKi, Aoi, Osaka-shi Osaka 532-8524 (JP); ICHINOMIYA, Mami, Osaka-shi Osaka 532-8524 (JP); MATSUDA, Takahiro, Osaka-shi Osaka 532-8524 (JP); TAKEDA, Ushio, Tokyo 140-0001 (JP)
(74) Representative: Mewburn Ellis LLP
(86) International application number: PCT/JP2024/020663
(87) International publication number: WO 2024/253152

(57) **Abstract**

An object of the present invention is to detect, with high sensitivity, soybean that may cause allergies contained in a food raw material, a product, or the like even if its amount is very small. The solution is a method for detecting soybean, comprising the steps of: extracting a protein from a sample; treating the extracted protein with a proteolytic enzyme to obtain an enzymatic digest; and analyzing the enzymatic digest and detecting at least one or more peptides selected from the group consisting of SEQ ID NOs: 1 to 3 using a mass spectrometer to qualitatively or quantitatively determine whether or not soybean protein is present in the sample.

## Description

### TECHNICAL FIELD

The present invention relates to a method for detecting soybean using a mass spectrometer, the method being capable of detecting, with high sensitivity, soybean that may cause food allergies contained in a food raw material, a product, or the like even if its amount is very small.

### BACKGROUND ART

Soybean (*Glycine max*), a plant of the genus *Glycine* in the family *Fabaceae,* is designated as "quasi-specific ingredients" recommended to be labeled as food-allergenic materials in Japan (The Notice Regarding Food Labeling Standards, Consumer Affairs Agency, No. 139 of March 30, 2015).

Foods that may cause allergies may be unintentionally incorporated in very small amounts in production, distribution, and processing stages. It is therefore important for providers of food raw materials or products to perform quality control on the presence or absence of their incorporation.

Examples of methods for testing the presence or absence of incorporation of particular foods include methods of detecting characteristic proteins using antigen-antibody reaction, such as ELISA, Western blot, and immunochromatography, and methods of detecting characteristic DNA nucleotide sequences by PCR, and the like.

Methods of detecting peptides derived from proteins characteristic of particular foods using a mass spectrometer have also been reported in recent years. Such techniques can quantify proteins in ingredients of interest and have the advantages that false positive reaction, which is prone to occur using antigen-antibody reaction, can be reduced; and a plurality of components can be detected at the same time.

For example, the present applicant has disclosed the following conventional technique regarding the detection of soybean.

### CITATION LIST

### PATENT LITERATURE

Patent Literature 1: Japanese Patent No. 4937306

However, this patent literature is directed to the detection of a gene, and other methods are possible.

### SUMMARY OF INVENTION

### TECHNICAL PROBLEM

Accordingly, an object of the present invention is to provide an analysis method using a mass spectrometer, which can specifically detect, with high sensitivity, soybean that may cause allergy from a food raw material or a product.

### SOLUTION TO PROBLEM

To attain the object, the present inventors have focused on the amino acid sequence of an allergen protein of soybean to be detected, and conducted diligent studies on a method that can specifically detect soybean with high sensitivity. As a result, the present inventors have found amino acid sequences characteristic of soybean and found that soybean can be specifically detected with high sensitivity by detecting these amino acid sequences, leading to the completion of the present invention. Specifically, the invention of the present application first relates to the following item.

### Item 1.

A method for detecting soybean, comprising the steps of: extracting a protein from a sample; treating the extracted protein with a proteolytic enzyme to obtain an enzymatic digest; and analyzing the enzymatic digest and detecting at least one or more peptides selected from the group consisting of SEQ ID NOs: 1 to 3 using a mass spectrometer to qualitatively or quantitatively determine whether or not soybean protein is present in the sample.

Next, a method for detecting the at least one or more peptides selected from the group consisting of SEQ ID NOs: 1 to 3 is preferably a method of analyzing the enzymatic digest by liquid chromatography-tandem mass spectrometry (LC-MS/MS) and monitoring at least one or more precursor-product ion pair transitions with specified m/z value associated with a specific amino acid sequence. Specifically, the invention of the present application relates to the following item 2.

### Item 2.

A method for detecting soybean, comprising the steps of: extracting a protein from a sample; treating the extracted protein with a proteolytic enzyme to obtain an enzymatic digest; and analyzing the enzymatic digest by liquid chromatography-tandem mass spectrometry (LC-MS/MS) and monitoring at least one or more precursor-product ion pair transitions with specified m/z value associated with a specific amino acid sequence selected from the group consisting of:
i) SEQ ID NO: 1, an m/z value of approximately 484/381, 484/643, or 484/294,
ii) SEQ ID NO: 2, an m/z value of approximately 713/213, 713/326, 713/515, 476/416, 476/515, or 476/213, and
iii) SEQ ID NO: 3, an m/z value of approximately 530/311, 530/147, or 530/424 to determine whether or not soybean protein is present in the sample.

Next, it is preferred to monitor two or more of the precursor-product ion pair transitions. Specifically, the invention of the present application relates to the following item 3.

### Item 3.

The method for detecting soybean according to item 2, wherein the method comprises the step of monitoring at least two or more precursor-product ion pair transitions with the specified m/z values associated with the specific amino acid sequences to qualitatively or quantitatively determine whether or not soybean protein is present in the sample.

### ADVANTAGEOUS EFFECTS OF INVENTION

The present invention provides effects that enable a soybean protein-derived peptide to be detected by LC-MS/MS analysis and enable a quality control test to be carried out on whether or not the soybean is incorporated or used in a test food raw material or a test food. The present invention can also contribute to the prevention of food allergy incidents, the inspection of a causative substance of manifested allergic symptoms, and the like.

### BRIEF DESCRIPTION OF DRAWINGS

[Figure 1] Figure 1 shows a peak of a soybean protein-derived peptide in a chromatogram obtained from a standard sample having a known soybean concentration.
[Figure 2] Figure 2 shows a calibration curve prepared by plotting an area of a soybean protein-derived peptide in a chromatogram obtained from a standard sample having a known soybean concentration, against a known soybean protein concentration in the standard sample.
[Figure 3] Figure 3 shows an exemplary chromatogram of an instant noodle sample containing no soybean.
[Figure 4] Figure 4 shows an exemplary chromatogram involving a soybean protein-derived peptide obtained from an instant noodle sample spiked with soybean protein.

### DESCRIPTION OF EMBODIMENTS

The present invention provides a method for detecting soybean protein incorporated in a very small amount in a test sample such as a food raw material or a processed food product. Specifically, the method comprises the steps of: extracting a protein from a test sample; treating the extracted protein with a proteolytic enzyme to obtain an enzymatic digest; and analyzing the enzymatic digest by LC-MS/MS to obtain a chromatogram of a peptide to be analyzed. Hereinafter, preferred forms of the method according to the present embodiment will be described.

The extraction of a protein from a test sample can be performed using a buffer solution containing a surfactant, a commercially available protein analysis kit, or the like.

The protein extract from the test sample is preferably further reduced and alkylated to block thiol groups.

The sample thus prepared is treated with a proteolytic enzyme. Examples of the proteolytic enzyme for use in the method of the present invention include trypsin, chymotrypsin, and the like. Trypsin is preferred. Treatment conditions can be appropriately selected depending on the type of the enzyme. The treatment with the enzyme degrades the protein to be detected to produce a plurality of peptides.

The obtained enzymatic digest is desirably analyzed by LC-MS/MS after removal of the surfactant, purification through a reverse-phase solid-phase column, or the like.

The peptide sequences to be analyzed in LC-MS/MS are as follows.

| | |
|---|---|
| SEQ ID NO: 1 | SQSDNFEYVSFK |
| SEQ ID NO: 2 | VLIVPQNFVVAAR |
| SEQ ID NO: 3 | FYLAGNQEQEFLK |

Various methods can be used as methods for detecting these soybean-derived peptides. The present invention utilizes a mass spectrometer.

Among them, a method using liquid chromatography is particularly preferred. Examples thereof include methods using LC-MS or LC-MS/MS.

The obtained enzymatic digest is particularly preferably analyzed by LC-MS/MS after removal of the surfactant, purification through a reverse-phase solid-phase column, or the like.

Soybean protein may be quantitatively analyzed by treating a standard sample having a known soybean-derived protein concentration in the same manner as in the test sample, analyzing the standard sample by LC-MS/MS, and preparing a calibration curve.

In the method for detecting soybean according to the present invention, the type of the test sample of interest is not particularly limited. Examples of the test sample include food raw materials, processed food products, and the like. Examples of the food raw material include separately produced food raw materials that do not intentionally contain soybean in food raw material production factories where soybean is handled. Examples of the processed food product include confectionery, noodles, powdered soups, liquid soups, hot-air dried or freeze-dried ingredients, various cooked food products containing these processed food products, and the like. Other examples thereof include separately produced processed food products that do not intentionally contain soybean in food production factories where soybean is handled. If a processed food product containing no soybean is produced after production of a processed food product containing soybean, elaborate cleaning work of a food production facility with the removal of soybean residues in mind is essential. A wipe sample from the production facility is also included in the test sample from the viewpoint of confirming the effectiveness of this cleaning work method as well as the presence or absence of soybean residues in the food production facility.

### Examples

Hereinafter, the present invention will be described in more detail with reference to Examples. However, the present invention should not be construed as being limited to these Examples. Appropriate changes or modifications may be made in the present invention without departing from the spirit of the present invention.

### Example 1

Analysis of standard sample having known soybean protein concentration

In order to verify the quantitativeness of the method for detecting soybean by LC-MS/MS according to the present invention, a standard sample having a known soybean protein concentration was analyzed, and a calibration curve was prepared.

A protein was extracted from soybean purchased from a store using MPEX PTS Reagents (60 mM SDC SLS/50 mM TEAB) (GL Sciences Inc.), and a total protein concentration was determined using Pierce BCA Protein Assay Kit (Thermo Fisher Scientific Inc.) to prepare a standard sample.

A 50 µg (in terms of the amount of the protein) aliquot of the prepared standard sample was collected into a 2.0 mL low-adsorption polypropylene tube, and the amount of the whole solution was adjusted to 700 µL by the addition of 1000 µg of egg-derived ovalbumin and 100 µg of bovine-derived albumin.

70 µL of 1 M TEAB and 28 µL of 1 M DTT were added to the tube, which was then left to stand at 75°C for 15 minutes and then left to stand at ordinary temperature for 30 minutes. Subsequently, 56 µL of an iodoacetamide solution adjusted to 1 M with distilled water was added to the tube, which was then left to stand at room temperature for 45 minutes in the dark, followed by the addition of 28 µL of 1 M DTT (reduction and alkylation).

10 µL of a bovine pancreas-derived trypsin solution prepared at 20 mg/mL in 0.1% formic acid was added to the tube, which was then left to stand overnight at 37°C to enzymatically digest the soybean standard sample.

The obtained enzymatic digest was rendered acidic by the addition of formic acid, and ethyl acetate was added thereto so that the surfactant contained in the extracted solution was removed by liquid-liquid partition. The removal operation was repeated three times.

The solution from which the surfactant was thus removed was concentrated with a centrifugal evaporator, and 0.1% formic acid was added to the concentrate, followed by purification using a C18 reverse-phase solid-phase extraction centrifugal column and a silica gel-based anion exchange solid phase.

The solution thus purified was evaporated to dryness in a centrifugal evaporator and dissolved in 0.1% formic acid containing 5% acetonitrile to prepare 1.25 to 10 µg/mL (in terms of the total soybean protein concentration) dilution series, which were then analyzed by LC-MS/MS.

### <LC-MS/MS apparatus>

LC unit: ExionLC AD system (SCIEX company)
MS/MS unit: QTRAP(R) 6500+ system (SCIEX company)

### <LC Conditions>

Analytical column: YMC-Triart C18, particle size: 3 µm, 100 × 2.1 mm id. (YMC company)
Column temperature: 40°C
Column flow rate: 0.3 mL/min
Eluent A: 0.1% formic acid; eluent B: acetonitrile containing 0.1% formic acid
Gradient: 0 min (B: 5%) → 16 min (B: 40%) → 18 min (B: 95%) → 23 min (B: 95%) → 23.1 min (B: 5%) → initialization

### <Mass spectrometry conditions>

Ionization: electrospray ionization
Polarity: positive
Spray voltage: 5500 V

Table 1 shows the sequences of soybean protein-derived peptide fragments to be detected and MRM transitions.

**[Table 1]**

| Peptide sequence to be detected | MRM transition | |
|---|---|---|
| | Q1 | Q3 |
| SQSDNFEYVSFK (SEQ ID NO: 1) | 484.2 | 381.2 |
| | 484.2 | 643.3 |
| | 484.2 | 294.2 |
| VLIVPQNFVVAAR (SEQ ID NO: 2) | 713.4 | 213.2 |
| | 713.4 | 326.2 |
| | 713.4 | 515.3 |
| | 476.0 | 416.3 |
| | 476.0 | 515.3 |
| | 476.0 | 213.2 |
| FYLAGNQEQEFLK (SEQ ID NO: 3) | 529.6 | 311.1 |
| | 529.6 | 147.1 |
| | 529.6 | 424.2 |

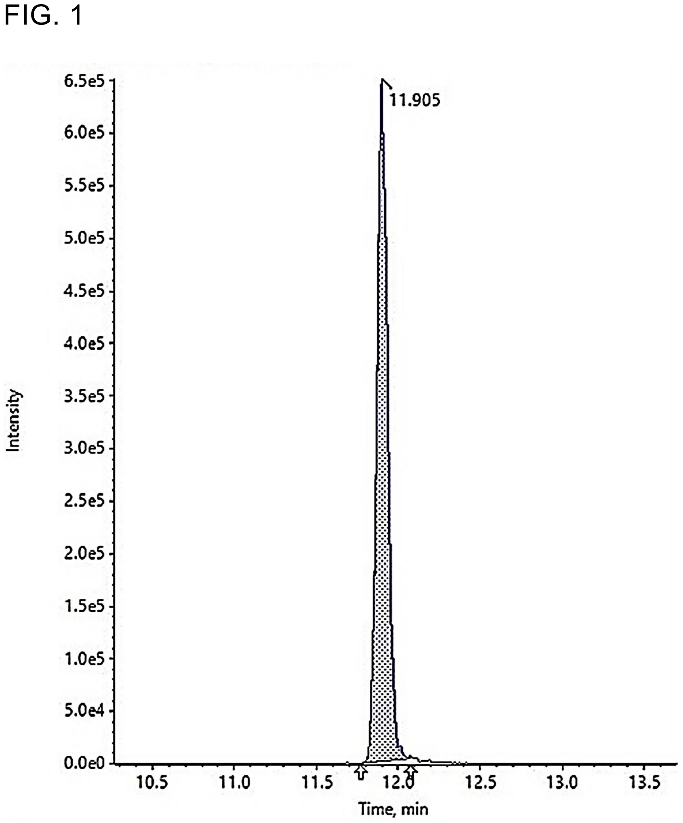

Figure 1 illustrates a chromatogram obtained by analyzing the standard sample having a total soybean protein concentration of 1.25 µg/mL (peptide sequence: VLIVPQNFVVAAR (SEQ ID NO: 2), Q1: 713.4, Q3: 213.2).

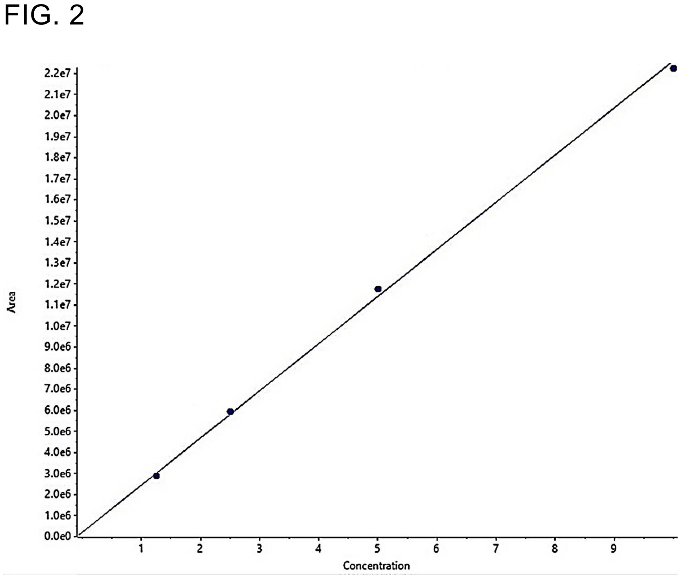

Figure 2 illustrates a calibration curve under the same detection conditions as in Figure 1. A favorable calibration curve with R2: 0.999 was obtained in a total soybean protein concentration range from 1.25 to 10 ppm.

### Example 2

Soybean protein spike test on processed food product

In order to verify the applicability of the method for detecting soybean by LC-MS/MS according to the present invention to a processed food product, an instant noodle ground product containing no soybean to which a soybean protein standard sample was added at 10 ppm was analyzed.

1 g of the instant noodle ground product sample containing no soybean was weighed into a 50 mL polypropylene centrifuge tube, to which the soybean protein standard sample used in Example 1 was added so as to attain a total soybean protein concentration of 10 ppm.

30 µL of ethylenediaminetetraacetic acid (EDTA) prepared at 100 mg/mL in a 1 N sodium hydroxide solution was added to the tube.

9 mL of the extracted solution used in Example 1 was added to the tube, which was then shaken overnight at 90 to 110 rpm to extract a protein.

The extract was centrifuged at 10,000 × g at 4°C for 30 minutes, and 700 µL of a supernatant was collected into a 2.0 mL low-adsorption polypropylene tube.

Subsequent operations were carried out in the same manner as in Example 1, and an undiluted final solution was analyzed by LC-MS/MS.

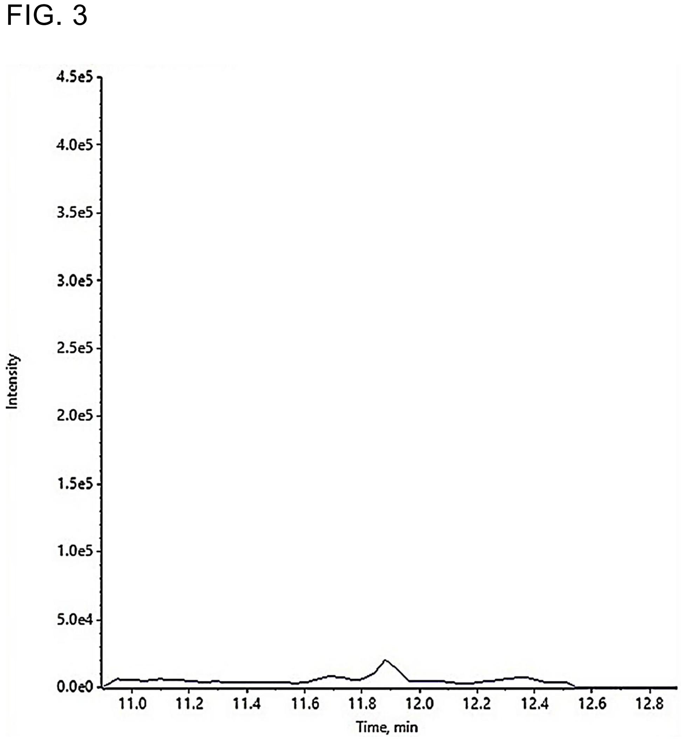

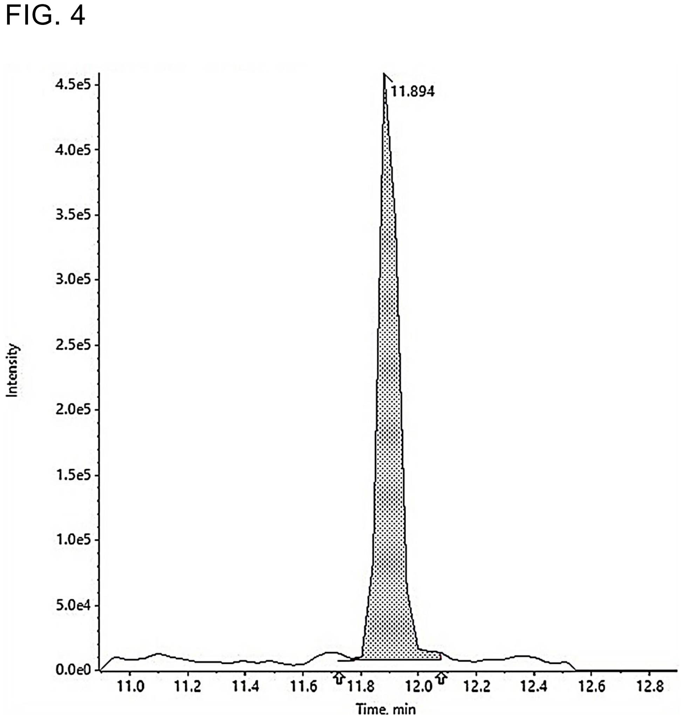

Figure 3 illustrates a chromatogram of the instant noodle ground product sample containing no soybean. Figure 4 illustrates a chromatogram of the sample supplemented with the soybean protein standard sample at a content of 10 ppm in the product (peptide sequence: VLIVPQNFVVAAR (SEQ ID NO: 2), Q1: 713.4, Q3: 213.2).

A peak of the analyte to be detected was observed only when the soybean protein standard sample was added.

## Claims

1. A method for detecting soybean, comprising the steps of: extracting a protein from a sample; treating the extracted protein with a proteolytic enzyme to obtain an enzymatic digest; and analyzing the enzymatic digest and detecting at least one or more peptides selected from the group consisting of SEQ ID NOs: 1 to 3 using a mass spectrometer to qualitatively or quantitatively determine whether or not soybean protein is present in the sample.

2. A method for detecting soybean, comprising the steps of: extracting a protein from a sample; treating the extracted protein with a proteolytic enzyme to obtain an enzymatic digest; and analyzing the enzymatic digest by liquid chromatography-tandem mass spectrometry (LC-MS/MS) and monitoring at least one or more precursor-product ion pair transitions with specified m/z value associated with a specific amino acid sequence selected from the group consisting of:
i) SEQ ID NO: 1, an m/z value of approximately 484/381, 484/643, or 484/294,
ii) SEQ ID NO: 2, an m/z value of approximately 713/213, 713/326, 713/515, 476/416, 476/515, or 476/213, and
iii) SEQ ID NO: 3, an m/z value of approximately 530/311, 530/147, or 530/424 to qualitatively or quantitatively determine whether or not soybean protein is present in the sample.

3. The method for detecting soybean according to claim 2, wherein the method comprises the step of monitoring at least two or more precursor-product ion pair transitions with the specified m/z values associated with the specific amino acid sequences to qualitatively or quantitatively determine whether or not soybean protein is present in the sample.
